# EUROPEAN PATENT APPLICATION

(11) **EP 4 830 638 A1**
(43) Date of publication of application: **28.10.2026**
(21) Application number: 25172377.1
(22) Date of filing: 24.04.2025
(51) Int. Cl.: A61B 8/00, G01C 25/00, G01S 7/52

(54) **METHODS AND SYSTEMS FOR AUTOMATED IMU CALIBRATION IN ULTRASOUND IMAGING GUIDANCE WORKFLOWS**

(71) Applicant: Koninklijke Philips N.V., 5656 AE Eindhoven (NL)
(72) Inventor: FLANNERY, Sean, Eindhoven (NL); BHARAT, Shyam, Eindhoven (NL)
(74) Representative: Philips Intellectual Property & Standards

(57) **Abstract**

A method (100) for calibrating an inertial measurement unit (IMU) sensor, comprising: detecting (120) that the integrated IMU sensor is connected to the ultrasound system; determining (130) whether the IMU sensor has or has not been previously calibrated; if the IMU sensor has been previously calibrated, then: retrieving (140) and applying stored calibration data from the calibration database; detecting (142) that the IMU sensor is at rest for a first resting period of time; and automatically calibrating (144) a gyroscope of the IMU sensor; if the IMU sensor has not been previously calibrated, then: instructing (150) a user to perform a calibration of the IMU sensor; automatically performing (152) the calibration of the IMU sensor, wherein the calibration generates an IMU sensor calibration bias and generates a calibrated IMU sensor; storing (154) the generated IMU sensor calibration bias in the calibration database.

## Description

### FIELD OF THE INVENTION

The present disclosure is directed generally to methods and systems for calibrating an IMU-equipped ultrasound probe with minimal user intervention during ultrasound image acquisition workflows.

### BACKGROUND OF THE INVENTION

Ultrasound technology has become a ubiquitous and indispensable tool in modern medicine. Its widespread adoption is due to its non-invasive nature, real-time imaging capabilities, and versatility in diagnosing and monitoring a variety of medical conditions. Ultrasound exams are routinely performed in obstetrics, cardiology, emergency medicine, and many other specialties, making it an essential component of contemporary healthcare practices. Its ability to deliver immediate, clear images without the risks associated with ionizing radiation further underscores its utility and widespread use in clinical settings worldwide.

Probe accuracy is critical to proper ultrasound imaging. The integration of inertial measurement unit (IMU) sensors into ultrasound probes enables real-time probe angle guidance to assist users in acquiring optimal imaging planes. This capability provides probe angle guidance to improve user accuracy and efficiency during ultrasound examinations. However, existing methods require a manual IMU calibration step at the start of each examination, where the user must position the probe in a fixed, known orientation to measure and correct for sensor biases. This step, while critical for ensuring accurate guidance, introduces workflow inefficiencies and user compliance challenges, particularly in high-volume screening environments where clinicians may perform 50-60 examinations per day.

### SUMMARY OF THE DISCLOSURE

There is thus a continued need for methods and systems that efficiently and accurately calibrate an ultrasound probe with minimal user involvement.

Various embodiments and implementations relate to the field of medical imaging and, more specifically, to systems and methods for improving the accuracy and usability of guidance technologies in devices equipped with inertial measurement unit (IMU) sensors. In particular, this disclosure addresses the challenges associated with IMU sensor calibration in applications such as ultrasound imaging, where probe angle guidance is critical for novice users. The methods and systems aim to reduce the burden of manual calibration while maintaining or improving the accuracy of probe angle guidance. By streamlining this process, the described innovations enhance the usability of ultrasound systems MA, thereby increasing their value proposition compared to competitors lacking advanced probe angle guidance capabilities. Furthermore, the methods and systems disclosed herein are broadly applicable to other IMU-based guidance applications. These advancements address a critical need for efficient, user-friendly calibration methods in IMU-integrated devices, enabling widespread adoption and improved performance across a range of applications.

Accordingly, various embodiments and implementations are directed to a method and system for efficiently and accurately calibrating an ultrasound probe with an integrated inertial measurement unit (IMU). The system determines, by comparing a unique identifier to a calibration database, whether a connected ultrasound probe has or has not been previously calibrated. If the ultrasound probe has been previously calibrated, then the system retrieves and applies stored accelerometer calibration data from the calibration database. The system detects that the ultrasound probe is at rest for a first resting period of time, and automatically calibrates a gyroscope of the IMU sensor to generate a fully calibrated ultrasound probe. However, if the ultrasound probe has not been previously calibrated, then the system instructs a user of the ultrasound probe to perform a calibration of the IMU sensor of the ultrasound probe. The system automatically performs a calibration of the IMU sensor of the ultrasound probe, and stores generated IMU sensor calibration bias in the calibration database.

According to an aspect, a method for calibrating an inertial measurement unit (IMU) sensor is provided. The method includes providing a system comprising an integrated IMU sensor; detecting, by the system using a unique identifier, that the integrated IMU sensor is connected to the ultrasound system; determining, by comparing the unique identifier to a calibration database, whether the IMU sensor has or has not been previously calibrated; if the IMU sensor has been previously calibrated, then: retrieving and applying stored calibration data from the calibration database; detecting that the IMU sensor is at rest for a first resting period of time; and automatically calibrating a gyroscope of the IMU sensor to generate a fully calibrated IMU sensor; if the IMU sensor has not been previously calibrated, then: instructing, via a user interface, a user of the IMU sensor to perform a calibration of the IMU sensor, comprising an instruction to place the IMU sensor on a flat surface for at least a first time period; automatically performing, by the system, the calibration of the IMU sensor, wherein the calibration generates an IMU sensor calibration bias and generates a calibrated IMU sensor; storing the generated IMU sensor calibration bias in the calibration database.

In accordance with an embodiment, the method further includes alerting, if the IMU sensor has been previously calibrated, a user that the IMU sensor has previously been calibrated.

In accordance with an embodiment, the method further includes alerting a user of that the calibrated IMU sensor has been generated.

In accordance with an embodiment, the method further includes alerting, if the IMU sensor has not been previously calibrated, that the IMU sensor has been successfully calibrated after generating the IMU sensor calibration bias.

In accordance with an embodiment, wherein the first time period is at least one second.

In accordance with an embodiment, the method further includes receiving ultrasound exam data from the fully calibrated IMU sensor.

In accordance with an embodiment, wherein when the IMU sensor has been previously calibrated, the method further includes determining that automatically calibrating the gyroscope of the IMU sensor to generate a calibrated IMU sensor was unsuccessful; instructing, via a user interface, a user to perform a one-time calibration of the IMU sensor, comprising an instruction to place the IMU sensor on a flat surface for at least a first time period; automatically performing, by the system, the calibration of the IMU sensor, wherein the calibration generates an IMU sensor calibration bias and generates a calibrated IMU sensor; and storing the generated IMU sensor calibration bias in the calibration database.

In accordance with an embodiment, the first resting period of time is at least 0.5 seconds.

In accordance with a second aspect, an ultrasound system is provided. The system includes: an ultrasound probe with an integrated inertial measurement unit (IMU) sensor; a calibration database; a user interface; and a processor configured to: (i) detect, using a unique identifier, that the ultrasound probe is connected to the ultrasound system; (ii) determine, by comparing the unique identifier to the calibration database, whether the ultrasound probe has or has not been previously calibrated; (iii) if the ultrasound probe has been previously calibrated, then: (1) retrieve and applying stored calibration data from the calibration database; (2) detect that the ultrasound probe is at rest for a first resting period of time; and (3) automatically calibrate a gyroscope of the IMU sensor to generate a fully calibrated ultrasound probe; (iv) if the ultrasound probe has not been previously calibrated, then: (1) instruct, via the user interface, a user of the ultrasound probe to perform a calibration of the IMU sensor of the ultrasound probe, comprising an instruction to place the ultrasound probe on a flat surface for at least a first time period; (2) automatically perform the calibration of the IMU sensor of the ultrasound probe, wherein the calibration generates an IMU sensor calibration bias and generates a calibrated ultrasound probe; and (3) store the generated IMU sensor calibration bias in the calibration database.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1 is a flowchart of a method for calibrating an IMU sensor, in accordance with an embodiment.
Fig. 2 is a schematic representation of an ultrasound system, in accordance with an embodiment.
Fig. 3 is a flowchart of a method for calibrating an ultrasound probe, in accordance with an embodiment.

### DETAILED DESCRIPTION OF EMBODIMENTS

The present disclosure describes various embodiments of a system and method configured to calibrate an ultrasound probe. More generally, Applicant has recognized and appreciated that it would be beneficial to more efficiently and accurately enable the calibration of an ultrasound probe with minimal user interaction. Thus, an ultrasound system determines, by comparing a unique identifier to a calibration database, whether a connected ultrasound probe has or has not been previously calibrated. If the ultrasound probe has been previously calibrated, then the system retrieves and applies stored calibration data from the calibration database. The system detects that the ultrasound probe is at rest for a first resting period of time, and automatically calibrates a gyroscope of the IMU sensor to generate a fully calibrated ultrasound probe. However, if the ultrasound probe has not been previously calibrated, then the system instructs a user of the ultrasound probe to perform a calibration of the IMU sensor of the ultrasound probe. The system automatically performs a calibration of the IMU sensor of the ultrasound probe, and stores generated IMU sensor calibration bias in the calibration database.

The embodiments and implementations disclosed or otherwise envisioned herein can be utilized with any system or process that may utilize or benefit from IMU calibration. The embodiments and implementations disclosed or otherwise envisioned herein can be utilized with any system that utilizes a calibrated ultrasound probe, including but not limited to Philips^{®} imaging modalities and devices (manufactured by Koninklijke Philips, N.V.), among other products. However, the disclosure is not limited to these devices or systems, and thus disclosure and embodiments disclosed herein can encompass any system that may utilize or benefit from IMU calibration, including systems other than ultrasound systems.

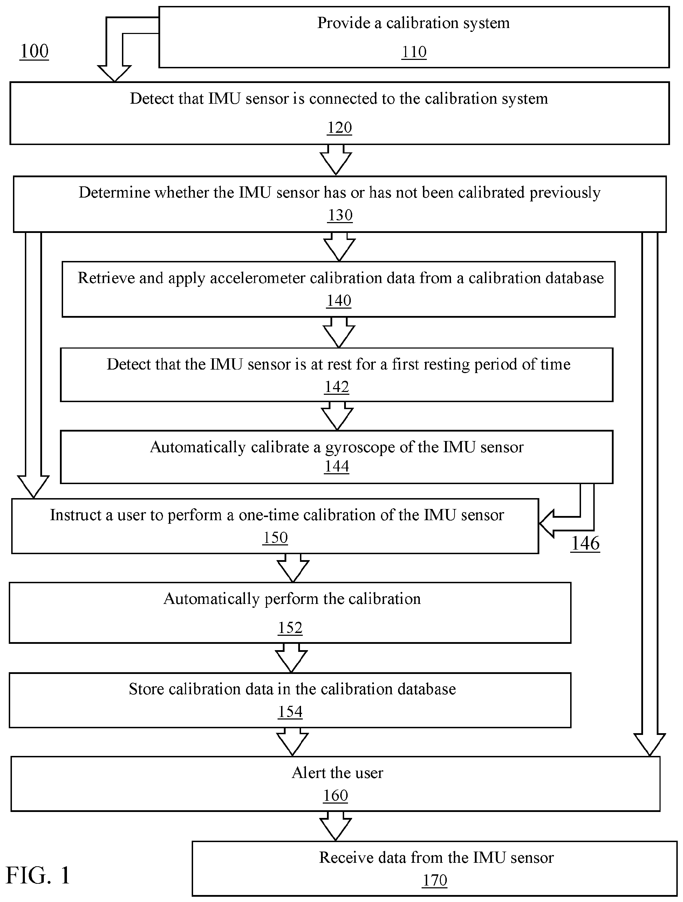

Referring to Fig. 1, in one embodiment, is a flowchart of a method 100 for calibrating, using a calibration system, an IMU sensor. The methods described in connection with the figures are provided as examples only, and shall be understood not to limit the scope of the disclosure. The calibration system can be any of the systems described or otherwise envisioned herein. The calibration system can be a single system or multiple different systems.

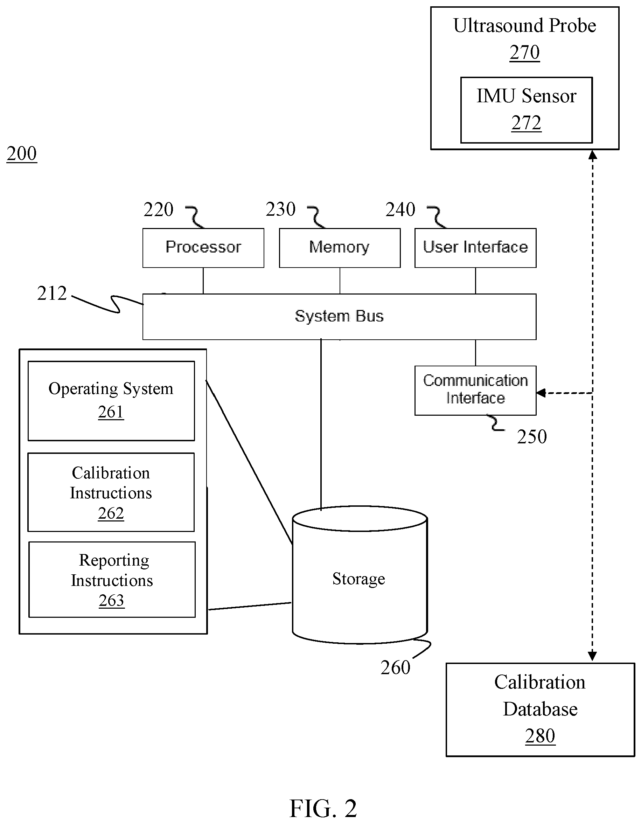

At step 110 of the method, an ultrasound system 200 is provided. Referring to an embodiment of an ultrasound system 200 as depicted in Fig. 2, for example, the system comprises one or more of a processor 220, memory 230, user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, ultrasound system 200 can be any of the systems described or otherwise envisioned herein. Other elements and components of the ultrasound system 200 are disclosed and/or envisioned elsewhere herein.

According to an embodiment, the ultrasound system 200 comprises or is in direct or indirect communication with an ultrasound probe 270. The ultrasound system can be, for example, an ultrasound system such as EPIQ^{®} system, Philips Lumify^{®}, and Philips Affinity^{®}, among many others. The images obtained using the ultrasound system may be obtained from a clinical provider or other individual. For example, the user of the ultrasound system may be an experienced sonographer, a first-time user of ultrasound, or any experience level in between. As another example, the ultrasound system comprises a handheld ultrasound probe connected to a mobile computing device such as a smartphone or tablet, among other mobile computing devices.

According to an embodiment, the ultrasound probe 270 comprises an inertial measurement unit (IMU) sensor 272. According to an embodiment, the IMU sensor is an electronic device that measures and reports acceleration, orientation, angular rates, and other gravitational forces. It can, for example, comprise 3 accelerometers and 3 gyroscopes, among other possible components. Thus, there is one component per axis for each of two or three axes: roll, pitch, and/or yaw. There are many other types of IMU sensors. The IMU sensor may be a standalone component, or it may be a component of another element of the ultrasound probe.

According to an embodiment, the IMU sensor 272 of the device (such as an ultrasound device although many other devices are possible) is integrated with the device. Notably, "integrated" can mean an internal component, wherein it may be integrated within an internal portion of the device. Alternatively, "integrated" can be attached to, wherein it may be attached to or otherwise permanently or temporarily connected to the device, either internally or externally.

According to an embodiment, the ultrasound system 200 comprises or is in direct or indirect communication with a calibration database 280. The calibration database 280 may comprise information about ultrasound probes, including a unique identifier (e.g., serial number) associated with an ultrasound probe, and information about whether or not the ultrasound probe has been calibrated. The information about whether or not the ultrasound probe has been calibrated can include, for example, when the ultrasound probe has been calibrated, one or more calibration biases for the ultrasound probe. The calibration database 280 may be local to system 200, and may optionally be a component of the system. The calibration database 280 may alternatively be remote to the system, and thus is in direct or indirection communication with the system.

According to another embodiment of Fig. 2, at step 110 of the method, a system 200 with an IMU sensor is provided. For example, IMUs are widely used in devices for tracking motion, orientation, and other physiological parameters. Examples of devices that incorporate IMU sensors include wearable devices, gait and motion analysis systems, prosthetic and orthotic devices, neurological and balance assessment tools, surgical tools, cardiac and respiratory monitors, stroke rehabilitation systems, and sports medicine devices, among many other devices. Referring to an embodiment of a system 200 with an IMU sensor as depicted in Fig. 2, for example, the system comprises one or more of a processor 220, memory 230, user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, system 200 can be any of the systems described or otherwise envisioned herein. Other elements and components of the system 200 are disclosed and/or envisioned elsewhere herein.

At step 120 of the method, system 200 detects that the IMU sensor is connected to the system. For example, an ultrasound system 200 detects that an ultrasound probe 270 is connected to the system. This can be accomplished in a wide variety of ways. According to an embodiment, the system automatically detects that the ultrasound probe 270 has been connected to the system. For example, this can be done automatically in response to connection of the ultrasound probe to the system. According to an embodiment, the system manually detects that the ultrasound probe 270 has been connected to the system. For example, a user can indicate to the system that an ultrasound probe has been connected.

According to an embodiment, when the ultrasound probe is connected to the ultrasound system, or when the system detects that the ultrasound probe is connected, the system can identify the ultrasound probe. For example, the probe may comprise a unique identifier, such as a serial number, RFID tag, or other identifier that comprises an identification of the specific probe. Thus, the system can both detect that the ultrasound probe is connected to the ultrasound system and can determine an identification of the probe using the unique identifier.

At step 130 of the system, the system determines whether the IMU sensor has previously been - or not been - calibrated. This can be accomplished in a variety of different ways. According to one embodiment, the system comprises a calibration database 280. The calibration database 280 may comprise information about ultrasound probes, including a unique identifier (e.g., serial number or other identifier) associated with an ultrasound probe, and information about whether or not the ultrasound probe has been calibrated. The information about whether or not the ultrasound probe has been calibrated can include, for example, when the ultrasound probe has been calibrated, one or more calibration biases for the ultrasound probe. The calibration database 280 may be local to system 200, and may optionally be a component of the system. The calibration database 280 may alternatively be remote to the system, and thus is in direct or indirection communication with the system.

By comparing the unique identifier to the calibration database, the system determines whether or not the IMU sensor (or, as described herein, an ultrasound probe comprising the IMU sensor) has previously been calibrated. If the device has been calibrated, and if that information has been provided to the calibration database, then the entry for the device will comprise calibration information.

According to an embodiment, the calibration information comprises one or more calibration biases. A calibration bias may be, for example, a correction to a deviation in measurement by the IMU sensor, as determined during a prior calibration protocol. For example, during the calibration protocol, calibration may systematically correct biases and/or misalignments in an IMU's accelerometers, gyroscopes, and/or magnetometers (if present). For gyroscope calibration, the IMU may be placed on a stable surface to measure and subtract the bias from the sensor's stationary outputs. Optionally, dynamic tests with known angular velocities can be used to fine-tune scale factors and detect axis misalignment. Similarly, for accelerometers, a six-position test can be conducted by orienting the IMU along its positive and negative axes to compare readings to the known gravitational acceleration (9.81 m/s²). These measurements can be used to correct biases, normalize scale factors, and align axes.

According to an embodiment, the calibration information may optionally include other information associated with the IMU sensor or the calibration (if the device has previously been calibrated), such as the date of calibration, location of calibration, and/or other information.

According to an embodiment, the comparison reveals that the IMU sensor has not been calibrated, or that the IMU sensor is not an entry in the calibration database, which indicates that the sensor has not been calibrated.

Thus, following step 130 of the method, the system has determined whether or not the IMU sensor has been previously calibrated.

For scenarios where the system determines that the ultrasound probe has been previously calibrated, then at step 140 of the method the system retrieves and applies stored calibration data from the calibration database 280. For example, the system can identify stored calibration data, such as calibration biases, during or following the previous determination step. The stored calibration data can thus be applied to the IMU sensor, the output of the sensor, and/or the workflow data obtained from the system, among other options.

Although the stored calibration data from the calibration database is applied to the system when the IMU sensor has previously been calibrated, according to an embodiment the gyroscope(s) of the IMU sensor may still need to be calibrated. Thus, at step 142 of the method, the system detects that the ultrasound probe is at rest for a first resting period of time. This can be accomplished, for example, by monitoring the IMU sensor data to determine that the ultrasound probe is at rest. The first resting period of time can be any period of time sufficient for calibration, including 0.5 seconds, 1.0 seconds, or more or less time depending on the calibration parameters of the IMU sensor.

At step 144 of the method, once the system has determined that the IMU sensor is at rest for a first resting period of time, the system automatically calibrates the gyroscope(s) of the IMU sensor to generate a fully calibrated IMU sensor. According to an embodiment, the system automatically calibrates the gyroscope(s) of the IMU sensor to generate a fully calibrated ultrasound probe.

According to an embodiment, the gyroscope can be calibrated in any way for calibrating a gyroscope. Calibrating a gyroscope in an IMU can comprise correcting for biases, scale factors, and misalignments to ensure accurate angular velocity measurements. While the device is at rest, the system can log raw gyroscope readings, and can calculate the average offset (bias) for each axis by averaging the recorded values and subtracting these biases from subsequent readings.

According to an embodiment, the fully calibrated IMU sensor (e.g., the fully calibrated ultrasound probe) is now ready for normal use.

Returning to step 130 of the method, the system alternatively determines that the IMU sensor has not been previously calibrated. For example, according to an embodiment, the comparison reveals that the IMU sensor has not been calibrated, or that the IMU sensor is not an entry in the calibration database, which indicates that the sensor has not been calibrated. According to another embodiment, the system may determine at step 130 that the calibration of the IMU sensor was at a past point in time, longer than a predetermined or preprogrammed or experimentally determined period to the present. In other words, the calibration was sufficiently long ago that a new calibration may be desired or necessary. According to an embodiment, this predetermined or preprogrammed or experimentally determined period may be based on time (e.g., 1 day ago, 1 week ago, 1 month ago, 1 year ago, or more or less than these time periods), or it may be based on usage (e.g., the IMU sensor has been used 5 times, 10 times, 50 times, 100 times, or more or less than this), among other foundations. Regardless of the exact method by which it is determined, at step 130 of the method the system determines that a calibration of the IMU sensor is necessary.

Accordingly, at step 150 of the method, the system instructs the user of the IMU sensor to perform a calibration of the sensor, which may be for example a calibration of an ultrasound probe. The instruction can be provided to the user via any method, including but not limited to via a user interface of the system 200. Thus, according to an embodiment, the system provides the notification via a user interface. The system may provide the notification to a user via any mechanism, including but not limited to a visual display, an audible notification, a page, or any other method of notification. The information may be communicated by wired and/or wireless communication to another device. For example, the system may communicate the information to a mobile phone, computer, laptop, wearable device, and/or any other device configured to allow display and/or other communication of the information.

The instruction to calibrate the IMU sensor can be any instruction sufficient to convey the need or desire for a calibration. It can be a required instruction, or an optional instruction, meaning that the user may be required to calibrate the IMU sensor or may be asked to optionally calibrate the IMU sensor. The instruction to calibrate the IMU sensor can comprise any information necessary to convey the calibration need and/or instructions. For example, the instruction may comprise one or more of: (1) an indication that the IMU sensor must be or should be or can be calibrated; (2) an indication of the last calibration of the IMU sensor, if any; (3) instructions for the calibration, including a command or request to place the IMU sensor on a flat surface for at least a first time period; and/or (4) any other information.

The command or request to place the IMU sensor on a flat surface for at least a first time period, conveyed to the user of the IMU sensor, enables the user to situate the IMU sensor such that a calibration can take place. The flat surface can be any surface, such as a table, a portion of an ultrasound system (for example), or any other surface. The first time period can be any time period necessary or sufficient to perform the calibration. According to an embodiment the first time period is 1 second, although shorter and longer first time periods are possible. The length of the time period may depend, for example, on the IMU sensor, the system, the conditions of the calibration, and/or any other parameters or conditions.
According to another embodiment in which the IMU sensor is integrated within an ultrasound probe, an ultrasound cart may be equipped with a probe holder that orients the probe vertically. Using the holder facilitates calibration because the orientation is known and one axis of the IMU aligns with gravity.

At step 152 of the method, the system automatically performs a calibration of the IMU sensor to generate a calibration bias and to generate a calibrated IMU sensor. The system automatically performs the calibration after determining that the IMU sensor is on the flat surface for at least the first period of time.

According to an embodiment, the system can automatically perform the calibration of the IMU sensor in any way known to calibrate an IMU sensor. For example, to calibrate an accelerometer of an IMU after placing the IMU on a flat, stable surface to ensure one axis is aligned with gravity, the system can obtain or record raw accelerometer readings for all three axes while the IMU remains stationary. The system can then identify the axis aligned with gravity, and compute the offsets for each axis by subtracting the expected acceleration values (±1 g or 0) from the raw measurements. These offsets can be applied to adjust future accelerometer readings, ensuring they are zero-centered when stationary. Repeat this process for multiple orientations if more comprehensive calibration is needed.
T hus, following the calibration, the system comprises an IMU sensor calibration bias and a calibrated IMU sensor. In the embodiment of an ultrasound probe, the system comprises a calibrated ultrasound probe.

At step 154 of the method, the system stores the generated IMU sensor calibration bias in the calibration database 280.

At step 160 of the method, the system provides information, an alert, an instruction, or other message to the user via the user interface of the system or via a user interface of another device or system. For example, the system can alert the user at 160 that the IMU sensor has previously been calibrated. The alert can comprise other information, including details or parameters of the calibration, including but not limited to the date of calibration. The system can alert the user at 160 that the gyroscope and/or accelerometer of the IMU sensor has been successfully calibrated. The system can alert the user at 160 that the attempted calibration of the gyroscope and/or accelerometer of the IMU sensor was not successful, optionally including instruction(s) for a corrective measure. Other alerts or messages or information are possible.

At step 170 of the method, the system can be used with the calibrated IMU sensor. The use of the system depends on the intended purpose of the system. In the embodiment of an ultrasound system, the ultrasound probe now comprises a calibrated IMU sensor and the user can obtain ultrasound imaging data using the calibrated ultrasound probe. The ultrasound imaging data can be obtained using known methods for obtaining ultrasound data using an ultrasound probe.

According to an optional embodiment of method 100 in Fig. 1, following a determination that the IMU sensor was previously calibrated and the system attempted to calibrate the gyroscope, the system determines at step 146 of the method that the automatic calibration of the gyroscope was unsuccessful and thus that the IMU sensor is not fully calibrated. Accordingly, after this determination, the system can optionally direct the user to proceed to step 150 of the method. The system can also, at step 160 of the method, instruct the user that the calibration was not successful, and provide instructions for next steps.

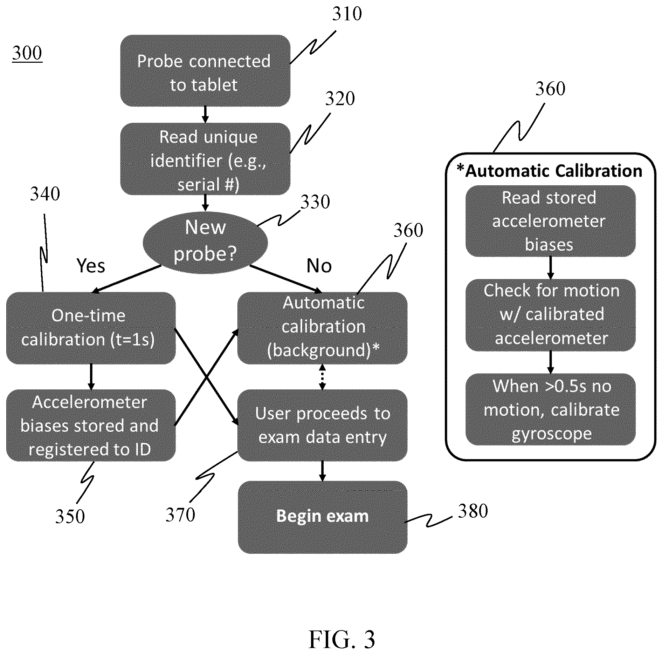

Referring to Fig. 3, in one embodiment, is a flowchart of a method 300 for calibrating, using an ultrasound system, an ultrasound probe. The methods described in connection with the figures are provided as examples only, and shall be understood not to limit the scope of the disclosure. The ultrasound system can be any of the systems described or otherwise envisioned herein. The ultrasound system can be a single system or multiple different systems.

At step 310 of the method, an ultrasound probe is attached to the ultrasound system. At step 320, the ultrasound system detects a unique identifier of the attached ultrasound probe. For example, the probe may comprise a unique identifier, such as a serial number, RFID tag, or other identifier that comprises an identification of the specific probe. Thus, the system can both detect that the ultrasound probe is connected to the ultrasound system and can determine an identification of the probe using the unique identifier.

At step 330, the system determines, using the unique identifier, whether the attached probe is a new probe (or, in other words, whether the probe has been previously calibrated. For example, according to one embodiment, the system comprises a calibration database 280. The calibration database 280 may comprise information about ultrasound probes, including a unique identifier (e.g., serial number or other identifier) associated with an ultrasound probe, and information about whether or not the ultrasound probe has been calibrated. The information about whether or not the ultrasound probe has been calibrated can include, for example, when the ultrasound probe has been calibrated, one or more calibration biases for the ultrasound probe. The calibration database 280 may be local to system 200, and may optionally be a component of the system. The calibration database 280 may alternatively be remote to the system, and thus is in direct or indirection communication with the system.

By comparing the unique identifier to the calibration database, the system determines whether or not the IMU sensor (or, as described herein, an ultrasound probe comprising the IMU sensor) has previously been calibrated. If the device has been calibrated, and if that information has been provided to the calibration database, then the entry for the device will comprise calibration information.

At step 340 of the method, the system determines that the probe is new or that the probe requires a calibration. Thus, at step 350 of the method, the system performs a calibration of the accelerometer of the IMU sensor of the ultrasound probe, and the calibration biases are stored in the calibration database or another database.

Alternatively, the system determines that the probe is not new or that the probe has previously been calibrated. Thus, at step 360 of the method, the system performs an auto-calibration of the gyroscope. This can comprise, for example, reading the accelerometer biases from the calibration database (i.e., the stored calibration biases), and checking for motion (i.e., finding a first motionless period of time), and then calibrating the gyroscope.

The system now comprises a fully calibrated IMU sensor and thus a fully calibrated ultrasound probe.

At step 370 of the method, the user can begin an ultrasound exam with the fully calibrated ultrasound probe. This can include, for example, entering patient or exam parameter information into the ultrasound system. Further, at step 380 of the method, the user begins the ultrasound exam.

Referring again to Fig. 2 is a schematic representation of a system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, system 200 comprises or is in direct or indirect communication with an IMU sensor 272, such as an IMU sensor of an ultrasound probe 270. According to an embodiment, the IMU sensor is an electronic device that measures and reports acceleration, orientation, angular rates, and other gravitational forces. The IMU sensor may be a standalone component, or it may be a component of another element of the ultrasound probe.

According to an embodiment, the ultrasound system 200 comprises or is in direct or indirect communication with a calibration database 280. The calibration database 280 may comprise information about ultrasound probes, including a unique identifier (e.g., serial number) associated with an ultrasound probe, and information about whether or not the ultrasound probe has been calibrated.

According to an embodiment, storage 260 of system 200 may store one or more algorithms, modules, and/or instructions to carry out one or more functions or steps of the methods described or otherwise envisioned herein. For example, storage 260 may comprise, among other instructions or data, calibration instructions 262 and/or reporting instructions 265.

According to an embodiment, calibration instructions 262 direct the system to perform the calibration of the IMU sensor accelerometer(s) and/or gyroscope(s). The IMU sensor accelerometer(s) and/or gyroscope(s) can be calibrated, pursuant to the calibration instructions 262, as described or otherwise envisioned herein, including but not limited to via the methods described in conjunction with Figs. 1 and 3.

According to an embodiment, reporting instructions 263 direct the system to provide the output of the system to a user, such as a clinician, via a user interface. The provided output can be any of the information as described or otherwise envisioned herein, including but not limited to calibration information, calibration instructions, success or failure of calibration, and/or any other information. The system may provide the information to a user via any mechanism, including but not limited to a visual display, an audible notification, a page, or any other method of notification. The information may be communicated by wired and/or wireless communication to another device. For example, the system may communicate the information to a mobile phone, computer, laptop, wearable device, and/or any other device configured to allow display and/or other communication of the information.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of.".

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for calibrating an inertial measurement unit (IMU) sensor, comprising:
providing (110) a system (200) comprising an integrated IMU sensor (272);
detecting (120), by the system using a unique identifier, that the integrated IMU sensor is connected to the ultrasound system;
determining (130), by comparing the unique identifier to a calibration database, whether the IMU sensor has or has not been previously calibrated;
if the IMU sensor has been previously calibrated, then:
retrieving (140) and applying stored calibration data from the calibration database;
detecting (142) that the IMU sensor is at rest for a first resting period of time; and
automatically calibrating (144) a gyroscope of the IMU sensor to generate a fully calibrated IMU sensor;
if the IMU sensor has not been previously calibrated, then:
instructing (150), via a user interface, a user of the IMU sensor to perform a calibration of the IMU sensor, comprising an instruction to place the IMU sensor on a flat surface for at least a first time period;
automatically performing (152), by the system, the calibration of the IMU sensor, wherein the calibration generates an IMU sensor calibration bias and generates a calibrated IMU sensor;
storing (154) the generated IMU sensor calibration bias in the calibration database.

2. The method of claim **1,** further comprising the step of alerting (160), if the IMU sensor has been previously calibrated, a user that the IMU sensor has previously been calibrated.

3. The method of claim **1,** further comprising the step of alerting (162) a user of that the calibrated IMU sensor has been generated.

4. The method of claim **1,** further comprising the step of alerting (160), if the IMU sensor has not been previously calibrated, that the IMU sensor has been successfully calibrated after generating the IMU sensor calibration bias.

5. The method of claim 1, wherein the first time period is at least one second.

6. The method of claim 1, further comprising the step of receiving (170) ultrasound exam data from the fully calibrated IMU sensor.

7. The method of claim 1, wherein when the IMU sensor has been previously calibrated, further comprising:
determining (146) that automatically calibrating the gyroscope of the IMU sensor to generate a calibrated IMU sensor was unsuccessful;
instructing (150), via a user interface, a user to perform a one-time calibration of the IMU sensor, comprising an instruction to place the IMU sensor on a flat surface for at least a first time period;
automatically performing (152), by the system, the calibration of the IMU sensor, wherein the calibration generates an IMU sensor calibration bias and generates a calibrated IMU sensor; and
storing (154) the generated IMU sensor calibration bias in the calibration database.

8. The method of claim 1, wherein the first resting period of time is at least 0.5 seconds.

9. An ultrasound system (200), comprising:
an ultrasound probe (270) with an integrated inertial measurement unit (IMU) sensor (272);
a calibration database (280);
a user interface (240); and
a processor (220) configured to: (i) detect, using a unique identifier, that the ultrasound probe is connected to the ultrasound system; (ii) determine, by comparing the unique identifier to the calibration database, whether the ultrasound probe has or has not been previously calibrated; (iii) if the ultrasound probe has been previously calibrated, then: (1) retrieve and applying stored calibration data from the calibration database; (2) detect that the ultrasound probe is at rest for a first resting period of time; and (3) automatically calibrate a gyroscope of the IMU sensor to generate a fully calibrated ultrasound probe; (iv) if the ultrasound probe has not been previously calibrated, then: (1) instruct, via the user interface, a user of the ultrasound probe to perform a calibration of the IMU sensor of the ultrasound probe, comprising an instruction to place the ultrasound probe on a flat surface for at least a first time period; (2) automatically perform the calibration of the IMU sensor of the ultrasound probe, wherein the calibration generates an IMU sensor calibration bias and generates a calibrated ultrasound probe; and (3) store the generated IMU sensor calibration bias in the calibration database.

10. The system of claim 9, wherein the user interface is configured to alert, if the ultrasound probe has been previously calibrated, a user of the ultrasound probe that the ultrasound probe has previously been calibrated.

11. The system of claim 9, wherein the user interface is configured to alert a user of the ultrasound probe that the calibrated ultrasound probe has been generated.

12. The system of claim 9, wherein the user interface is configured to alert, if the ultrasound probe has not been previously calibrated, that the ultrasound probe has been successfully calibrated after generating the IMU sensor calibration bias.

13. The system of claim 9, wherein the first time period is at least one second.

14. The system of claim 9, wherein the processor is further configured to receive ultrasound exam data from the fully calibrated ultrasound probe.

15. The system of claim 9, wherein when the ultrasound probe has been previously calibrated, the processor is further configured to:
determine that automatically calibrating the gyroscope of the IMU sensor to generate a fully calibrated ultrasound probe was unsuccessful;
instruct, via a user interface, a user of the ultrasound probe to perform a calibration of the IMU sensor of the ultrasound probe, comprising an instruction to place the ultrasound probe on a flat surface for at least a first time period;
automatically perform, by the ultrasound probe, the calibration of the IMU sensor of the ultrasound probe, wherein the calibration generates an IMU sensor calibration bias and generates a calibrated ultrasound probe; and
store the generated IMU sensor calibration bias in the calibration database.
